# EUROPÄISCHE PATENTSCHRIFT

(11) **EP 1 446 377 B1**
(45) Veröffentlichungstag und Bekanntmachung des Hinweises auf die Patenterteilung: **09.11.2005**
(21) Anmeldenummer: 02803007.0
(22) Anmeldetag: 11.11.2002
(51) Int. Cl.: C07C 209/60

(54) **VERFAHREN ZUR HERSTELLUNG VON AMINEN DURCH OLEFINAMINIERUNG IN GEGENWART UNGESÄTTIGTER STICKSTOFFVERBINDUNGEN**
METHOD FOR PRODUCING AMINES BY MEANS OF OLEFIN AMINATION IN THE PRESENCE OF UNSATURATED NITROGEN COMPOUNDS
PROCEDE POUR LA PRODUCTION D'AMINES PAR AMINATION D'OLEFINES EN PRESENCE DE COMPOSES AZOTES INSATURES

(30) Priorität: 12.11.2001 DE 10155523
(43) Veröffentlichungstag der Anmeldung: 18.08.2004
(73) Patentinhaber: BASF Aktiengesellschaft, 67056 Ludwigshafen (DE)
(72) Erfinder: BÖHLING, Ralf, 64347 Griesheim (DE); STEINBRENNER, Ulrich, 67435 Neustadt (DE); FUNKE, Frank, 67067 Ludwigshafen (DE); DIER, Reinhard, 66629 Freisen (DE)
(74) Vertreter: Isenbruck, Günter
(86) Internationale Anmeldenummer: PCT/EP2002/012582
(87) Internationale Veröffentlichungsnummer: WO 2003/042156

(56) Entgegenhaltungen:
- EP-A- 1 157 983
- DE-A- 2 161 750
- DE-A- 10 041 676
- DE-A- 10 046 608
- DE-B- 1 034 643
- US-A- 2 984 687
- US-A- 3 513 200
- US-A- 4 186 148
- US-A- 4 533 751
- PATENT ABSTRACTS OF JAPAN vol. 003, no. 050 (C-044), 27. April 1979 (1979-04-27) & JP 54 024836 A (MITSUI PETROCHEM IND LTD), 24. Februar 1979 (1979-02-24)
- PATENT ABSTRACTS OF JAPAN vol. 003, no. 011 (C-035), 30. Januar 1979 (1979-01-30) & JP 53 135912 A (NISSAN CHEM IND LTD), 28. November 1978 (1978-11-28)

## Beschreibung

Die Erfindung betrifft ein Verfahren zur Herstellung von Alkylaminen durch Umsetzung von Alkenen mit Aminen in Anwesenheit eines Metallhydrids oder Metalls als Katalysator.

Die Substanzklasse der Amine ist sowohl in der Natur als auch in der chemischen Industrie von überaus großer Bedeutung. Die Amine mit großer industrieller Bedeutung sind häufig einfach aufgebaute aliphatische Verbindungen, die für eine Vielzahl von industriellen Anwendungen in sehr großen Mengen benötigt werden. Als Beispiele seien die Weiterverarbeitung der Amine zu Pharmaka, Pflanzenschutzmitteln, Farb- und Kunststoffen sowie ihr Einsatz als Hilfsmittel in der Gummi-, Textil- und Papierindustrie genannt. Für sehr viele Bereiche der organischen Chemie, d. h. sowohl für die Laborsynthese von Naturstoffen oder biologisch aktiven Substanzen als auch für die industrielle Produktion von Aminen, sind möglichst einfache, flexible und vor allem kostengünstige Herstellmethoden für Amine daher von enormer Bedeutung.

Klassische Methoden zur Herstellung von Aminen sind sowohl im Labor als auch in der industriellen Produktion die Umwandlung von Alkoholen oder Alkylhalogeniden in Amine, die reduktive Aminierung von Carbonylverbindungen, die Reduktion von Nitrilen oder Aziden, Michael-Addition von Aminen bzw. Ammoniak an aktivierte Alkene sowie die Ritterreaktion. Allen aufgeführten Methoden ist gemeinsam, dass die verwendeten Ausgangsmaterialien bereits relativ hoch veredelt, teilweise schwer zugänglich bzw. handhabbar und somit meist teuer sind.

Diese Synthesewege könnten drastisch verkürzt werden, wenn es gelänge, effektive, generell anwendbare Hydroaminierungsverfahren für Alkene zu entwickeln. Es sind bereits einige Verfahren zur Hydroaminierung von Alkenen bekannt.

US 2,501,556 sowie B. W. Howk et al., J. Am. Chem. Soc. 76 (1954), 1899ff bis 1902 betreffen die Aminierung von nicht aktivierten Olefinen mit einem Amin oder NH₃ unter Einsatz von Alkalimetallen, Alkalimetallhydriden oder Alkalimetallamiden als Katalysatoren. Zur Erzielung akzeptabler Ausbeuten und Umsätze sind Temperaturen von etwa 175 bis 200°C und hohe Drucke von über 400 atm. erforderlich. Mit sinkendem Druck fallen Ausbeute und Umsatz drastisch ab.

Des Weiteren sind Verfahren bekannt, die hoch empfindliche und teure Katalysatoren, z. B. Lanthanoidverbindungen, verwenden bzw. Verfahren, die lediglich zur Herstellung spezieller Amine geeignet sind.

US-A 4,533,751 betrifft ein Verfahren zur Herstellung von 1,3-Dienaminen aus Myrcen und substituierten Myrcenen durch Umsetzung mit einem sekundären Amin in Anwesenheit einer katalytischen Menge des korrespondierenden Alkalimetallamids, das aus einem Alkalimetallhydrid oder einem Alkalimetall des sekundären Amins gebildet wird.

Eine breit anwendbare Methode für die Hydroaminierung von Alkenen steht daher bisher nicht zur Verfügung.

Aufgabe der vorliegenden Erfindung ist es daher, ein wirtschaftliches Verfahren zur Herstellung von Alkylaminen bereitzustellen, das mit leicht erhältlichen bzw. leicht handhabbaren, preiswerten Ausgangsprodukten auskommt, hohe Raum-Zeit-Ausbeuten und Selektivitäten ermöglicht sowie an preiswerten Katalysatorsystemen durchführbar ist.

Die Lösung der Aufgabe geht aus von einem Verfahren zur Herstellung von Alkylaminen (Produktaminen) durch Umsetzung von Alkenen mit Aminen (Eduktaminen) in Anwesenheit eines Metallhydrids oder Metalls als Katalysator.

Das erfindungsgemäße Verfahren ist dann dadurch gekennzeichnet, dass die Umsetzung in Anwesenheit eines Cokatalysators erfolgt, der eine ungesättigte Stickstoffverbindung in Form einer cyclischen oder offenkettigen Imin- oder der tautomeren Enaminverbindung ist.

Mit dem Verfahren der vorliegenden Anmeldung wird erstmals ein einstufiger Zugang zu Aminen aus Alkenen mit einer breiten Anwendbarkeit bereitgestellt, unter Bedingungen, die großtechnisch akzeptabel sind. Dabei kann von preiswerten und gut verfügbaren Alkenen ausgegangen werden und der fehlende Zwangsanfall von Nebenprodukten macht die Reaktion außerdem aus ökologischer Sicht interessant. Die Anwendung des vorliegenden Verfahrens bei der industriellen Produktion von Aminen führt somit zu enormen Einsparungen.

Bei den gemäß dem vorliegenden Verfahren eingesetzten Alkenen handelt es sich bevorzugt um nichtaktivierte Alkene. Unter nichtaktivierten Alkenen sind gemäß der vorliegenden Anmeldung solche Alkene zu verstehen, deren olefinische Doppelbindung nicht in Konjugation mit anderen olefinischen oder aromatischen Doppelbindungen bzw. Heteroatomen steht. Die Hydroaminierung dieser Alkene ist besonders schwierig, da ein nukleophiler Angriff der Aminogruppe auf die olefinische Doppelbindung nicht durch Aktivierung der Doppelbindung durch Konjugation oder Heteroatome erleichtert wird. Aus nichtaktivierten Alkenen, z. B. Ethen oder Propen, gewonnene Amine sind jedoch von besonderem Interesse.

Grundsätzlich sind beliebige nichtaktivierte Alkene in dem erfindungsgemäßen Verfahren geeignet. Bevorzugt sind dabei Alkene, die keine weiteren funktionellen Gruppen tragen. Besonders bevorzugt werden nichtaktivierte Alkene mit 2 bis 20 Kohlenstoffatomen eingesetzt, wobei die Alkene verzweigt, unverzweigt oder cyclisch sein können. Ganz besonders bevorzugt weisen die Alkene 2 bis 6 Kohlenstoffatome auf, wie Ethen, Propen, n-Buten mit einer Doppelbindung an der Position 1 oder 2, iso-Buten, n-Penten mit einer Doppelbindung in Position 1 oder 2, iso-Penten und n-Hexen mit einer Doppelbindung in Position 1, 2 oder 3 sowie iso-Hexen. Ganz besonders bevorzugt werden Ethen und Propen eingesetzt.

Bei den eingesetzten Aminen (im Folgenden Eduktamine genannt) handelt es sich um Alkylamine, Alkylarylamine oder Arylamine wie Anilin. Bevorzugt sind primäre oder sekundäre Alkylamine, besonders bevorzugt sekundäre Alkylamine. Die Alkylgruppe der Alkylamine kann dabei verzweigt, unverzweigt oder cyclisch sein. Bevorzugt werden Alkylamine eingesetzt, deren Alkylgruppen gesättigt sind und keine funktionellen Gruppen enthalten. Besonders bevorzugt weisen die Alkylgruppen 1 bis 20 Kohlenstoffatome, ganz besonders bevorzugt 1 bis 6 Kohlenstoffatome auf, d. h. es handelt sich ganz besonders bevorzugt um Methyl-, Ethyl-, n-/iso-Propyl-, n-/iso-/tert.-Butyl-, n-Pentyl- oder verzweigte Pentyl-, n-Hexyl- oder verzweigte Hexylreste. Ganz besonders bevorzugt werden Alkylamine eingesetzt, deren Alkylreste 1 bis 4 Kohlenstoffatome tragen. Werden sekundäre Alkylamine eingesetzt, so können die Alkylgruppen der Alkylamine gleich oder verschieden sein. Ganz besonders bevorzugt werden Ethylamin, Diethylamin, Isopropylamin, Diisopropylamin und Dimethylamin eingesetzt.

Bei den durch das erfindungsgemäße Verfahren hergestellten Alkylaminen (oder gegebenenfalls Alkylarylaminen) (im Folgenden Produktamine genannt) handelt es sich bevorzugt um sekundäre oder tertiäre Alkylamine (oder gegebenenfalls Alkylarylamine). Dabei können, in Abhängigkeit von den eingesetzten Aminen, die Alkylgruppen gleich oder verschieden sein. Bevorzugte Alkylreste entsprechen denen der eingesetzten Amine. Ganz besonders bevorzugt hergestellte Amine sind Triethylamin, Diisopropylamin, Ethylisopropylamin sowie Ethyldimethylamin. Diese werden bevorzugt durch die in der folgenden Tabelle aufgeführten Umsetzungen erhalten:

| Eduktamin | Alken | Produktamin |
|---|---|---|
| H₂NEt,HNEt₂ | Ethen | NEt₃, HNEt₂ |
| H₂NⁱPr | Propen | HN(ⁱPr)₂ |
| HN(ⁱPr)₂ | Ethen | EtN(ⁱPr)₂ |
| HMe₂N | Ethen | EtNMe₂ |

Als Metallhydride werden in dem erfindungsgemäßen Verfahren im Allgemeinen Alkalimetallhydride wie Lithiumhydrid, Natriumhydrid, Kaliumhydrid, Rubidiumhydrid und Cäsiumhydrid eingesetzt. Bevorzugt werden Lithiumhydrid, Natriumhydrid, Kaliumhydrid eingesetzt, besonders bevorzugt Natriumhydrid und Kaliumhydrid und ganz besonders bevorzugt Natriumhydrid. Vorzugsweise werden Hydride eingesetzt, die in gesättigten Kohlenwasserstoffatomen als Pasten oder Suspensionen vorliegen. Ganz besonders bevorzugt werden Pasten oder Suspensionen der Hydride in Paraffinöl eingesetzt. Insbesondere bevorzugt ist der Einsatz technischer Hydride, wie sie z. B. von den Firmen Callery, Chemmetall, Degussa-Hüls oder Metallchemie vertrieben werden. Es handelt sich dabei um Pasten in gesättigten Kohlenwasserstoffen, abgepackt in löslichen Kunststoffbeuteln. Sie sind in der Regel durch Alkalimetall und/oder Alkalihydroxid verunreinigt.

Als Metalle werden in dem erfindungsgemäßen Verfahren im Allgemeinen Alkalimetalle wie Li, Na, K, Rb oder Cs, bevorzugt Li, Na oder K, besonders bevorzugt Na oder K, ganz besonders bevorzugt Na eingesetzt.

Es können auch Legierungen der Alkalimetalle untereinander eingesetzt werden. Eine Vorreinigung der Alkalimetalle vor dem Einsatz in die Reaktion ist nicht notwendig. So kann technisches Alkalimetall, wie es z.B. von den Firmen Métaux-Spéciaux, Chemmetall, Callery Chem. Comp., PA oder Alkali Metals Ltd., Hyderabad, Indien angeboten wird, eingesetzt werden. Das Alkalimetall ist in der Regel verunreinigt durch Hydroxide, Oxide, Erdalkalimetalle, Halogenide und ― im Falle des Lithiums ― Nitride.

Die als Katalysatoren eingesetzten Hydride und Metalle stellen preiswerte, leicht zugängliche und leicht handhabbare Katalysatoren dar, die eine breite, preiswerte und auch industriell handhabbare Durchführung des erfindungsgemäßen Verfahrens ermöglichen.

Weiterhin bevorzugt ist der Einsatz von Hydriden, die aus dem Zerfall von Alkaliamiden oder Alkaliorganylen stammen. Die Bildung des Hydrids kann dabei im Reaktor in-situ, bevorzugt bei Temperaturen von >50°C, erfolgen.

Werden Hydride eingesetzt, die als Paste oder Suspension in gesättigten Kohlenwasserstoffen vorliegen, so können diese Hydride direkt eingesetzt werden oder vor dem Einsatz in dem erfindungsgemäßen Verfahren gewaschen werden, um das Paraffinöl weitgehend zu entfernen. Solche Waschvorgänge sind dem Fachmann geläufig. In einer bevorzugten Ausführungsform werden die Hydride durch Waschen mit dem Eduktamin von dem gesättigten Kohlenwasserstoff befreit. Das Waschen des eingesetzten Hydrids kann dabei direkt im Reaktionsgefäß oder in einem anderen Gefäß und anschließendes Umfüllen erfolgen.

Es wurde gefunden, dass die Umsetzung von Alkenen mit Aminen (Eduktaminen) zu Alkylaminen (oder gegebenenfalls zu Alkylarylaminen) (Produktaminen), die bevorzugt sekundär oder tertiär sind, mit Metallhydriden oder Metallen als Katalysatoren dann mit besonders hohen Raum-Zeit-Ausbeuten verläuft, wenn ein Cokatalysator eingesetzt wird, dessen Acidität höher als die des Amins ist. Bevorzugt ist die Acidität jedoch nicht so hoch und die Nucleophilie des konjugierten Anions so gering, daß eine Addition an Ethylen unwahrscheinlich wird. Ohne an eine Theorie gebunden zu sein, wird davon ausgegangen, dass die Acidität der Eduktamine allein nicht ausreichend ist, um eine Deprotonierung durch das als Katalysator eingesetzte Metallhydrid oder Metall quantitativ zu ermöglichen, besonders, wenn der entstehende Wasserstoff nicht aus dem System entfernt wird. Der eingesetzte Cokatalysator ist durch das als Katalysator eingesetzte Metallhydrid oder Metall leichter deprotonierbar und der so erhaltene Metallkomplex ist in der Lage, an das Alken zu addieren. Dadurch wird eine Metall-Kohlenstoff-Bindung erhalten, die leicht durch das Eduktamin protoniert werden kann, so dass ein Metallkomplex aus dem Metall des eingesetzten Metallhydrids und dem Eduktamin gebildet wird. Dieser kann nun an das eingesetzte Alken addieren und den Reaktionszyklus fortsetzen.

Als Cokatalysatoren werden cyclische oder offenkettige Imin- oder die tautomeren Enaminverbindungen eingesetzt.

Bevorzugt eingesetzte offenkettige Stickstoff-Verbindungen sind ausgewählt aus offenkettigen Imin- oder den tautomeren Enaminverbindungen der allgemeinen Formel (I) bzw. (Ia) 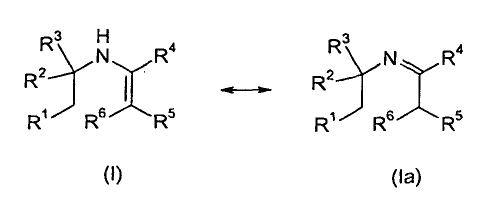 worin die Reste R¹ bis R⁶ unabhängig voneinander Wasserstoff oder Alkylreste bedeuten, die verzweigt oder unverzweigt sein und/oder durch ein oder mehrere Stickstoffatome unterbrochen sein können. Bevorzugt sind R¹ bis R⁶ C₁ - C₂₀ -Alkylreste, besonders bevorzugt C₁ - C₄ -Alkylreste. Geeignete Alkylreste sind beispielsweise Methyl, Ethyl, n-bzw. iso-Propyl, n- bzw. iso- bzw. tert.-Butyl.

R¹ bis R⁶ können auch unabhängig voneinander Cycloalkylreste sein, die durch die oben genannten funktionellen Gruppen oder durch Alkyl-, Alkenyl- oder Alkinylgruppen substituiert und/oder durch ein oder mehrere Stickstoffatome unterbrochen sein können. Bevorzugt eingesetzte Cycloalkylreste weisen 3 bis 12 Kohlenstoffatome - gegebenenfalls teilweise durch Stickstoffatome ersetzt - in ihrem Ring auf, besonders bevorzugt 5 oder 6 Kohlenstoffatome. Ganz besonders bevorzugt sind die Cycloalkylreste unsubstituiert. Besonders geeignete Cycloalkylreste sind beispielsweise Cyclopentyl und Cyclohexyl.

Es ist weiterhin möglich, dass die Reste R¹ bis R⁶ unabhängig voneinander Alkenylreste oder Alkinylreste sind, die eine oder mehrere Mehrfachbindungen, bevorzugt 1 bis 4 Mehrfachbindungen aufweisen. Die Alkenyl- bzw. Alkinylreste können entsprechend den Alkylresten substituiert oder durch ein oder mehrere Stickstoffatome unterbrochen sein.

Es ist auch möglich, dass zwei der Reste R¹ bis R⁶ gemeinsam einen Ring bilden, der wiederum mit Alkyl-, Alkenyl- oder Alkinylgruppen substituiert sein kann.

Besonders bevorzugt sind die Reste R¹ bis R⁶ unabhängig voneinander Wasserstoff, Methyl oder Ethyl, wobei die Reste R¹ bis R⁶ ganz besonders bevorzugt jeweils Wasserstoff sind.

Als cyclische ungesättigte Sickstoff-Verbindungen sind sowohl cyclische Enamine als auch N-heterocyclische Verbindungen in Form von Iminen oder Enaminen geeignet. Bevorzugt eingesetzte cyclische Enamine weisen einen einen C₄ bis C₈-Kohlenstoffring, besonders bevorzugt einen C₄ bis C₆-Kohlenstoffring auf. Dieser Kohlenstoffring ist mindestens einfach ungesättigt und trägt mindestens eine Aminogruppe, die mindestens ein Wasserstoffatom aufweist. Je nach Ringgröße kann der Kohlenstoffring auch zwei oder mehr Doppelbindungen tragen. Bevorzugt werden cyclische Enamine eingesetzt, die nicht weiter konjugiert sind. Der Kohlenstoffring kann durch einen oder - in Abhängigkeit von der Ringgröße - mehrere Reste, neben der Aminogruppe, substituiert sein. Geeignete Reste entsprechen denen bereits vorstehend für R¹ bis R⁶ genannten Resten, mit Ausnahme von Wasserstoff (im Rahmen der Substitution dieses Kohlenstoffrings mit Resten wird eine Substitution mit Wasserstoff als kein Rest aufgefasst). Bevorzugte Reste sind Alkylreste, die verzweigt oder unverzweigt sein können und 1 bis 6, besonders bevorzugt 1 bis 4 Kohlenstoffatome aufweisen. Die Anzahl der Reste ist abhängig von der Ringgröße, wobei kein bis 3 Reste bevorzugt sind und der Kohlenstoffring besonders bevorzugt keinen oder einen, ganz besonders bevorzugt keinen Rest trägt.

Geeignete cyclische ungesättigte Sickstoff-Verbindungen sind beispielsweise: 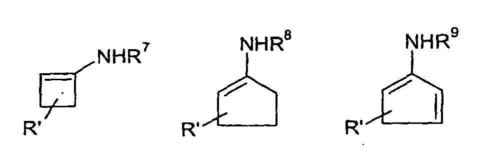 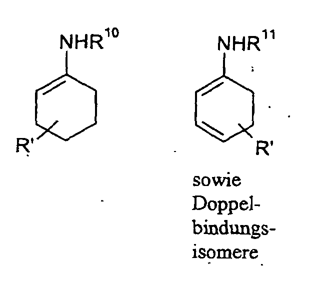 darin bedeuten R⁷ bis R¹² Wasserstoff oder einen der für R¹ bis R⁶ genannten Reste. Bevorzugt sind R⁷ bis R¹² Wasserstoff, Methyl- oder Ethylreste, ganz besonders bevorzugt Wasserstoff oder Ethylreste.

R' kann ein einziger oder mehrere Substituenten am Kohlenstoffring sein, wobei die maximale Anzahl der Reste R' der Zahl der Wasserstoffatome der Ringkohlenstoffatome entspricht. Bevorzugt ist die Anzahl der Reste R' 0 bis 3, besonders bevorzugt 0 bis 1, ganz besonders bevorzugt 0, das heißt, alle Kohlenstoffatome des Kohlenstoffrings sind mit Wasserstoff substituiert, mit Ausnahme eines Kohlenstoffatoms, das die Aminogruppe trägt. Geeignete Reste R' sind unabhängig voneinander Alkylreste, die verzweigt oder unverzweigt sein und/oder durch ein oder mehrere Stickstoffatome unterbrochen sein können. Bevorzugt sind die Reste R' C₁ - C₂₀ -Alkylreste, besonders bevorzugt C₁ - C₄ - Alkylreste. Geeignete Alkylreste sind beispielsweise Methyl, Ethyl, n- bzw. iso-Propyl, n-bzw. iso- bzw. tert.-Butyl.

Die Reste R' können auch unabhängig voneinander Cycloalkylreste sein, die durch die oben genannten funktionellen Gruppen oder durch Alkyl-, Alkenyl- oder Alkinylgruppen substituiert und/oder durch ein oder mehrere Stickstoffatome unterbrochen sein können. Bevorzugt eingesetzte Cycloalkylreste weisen 3 bis 12 Kohlenstoffatome - gegebenenfalls teilweise durch Stickstoffatome ersetzt - in ihrem Ring auf, besonders bevorzugt 5 oder 6 Kohlenstoffatome. Ganz besonders bevorzugt sind die Cycloalkylreste unsubstituiert. Besonders geeignete Cycloalkylreste sind beispielsweise Cyclopentyl und Cyclohexyl.

Es ist weiterhin möglich, dass die Reste R' unabhängig voneinander Alkenylreste oder Alkinylreste sind, die eine oder mehrere Mehrfachbindungen, bevorzugt 1 bis 4 Mehrfachbindungen aufweisen. Die Alkenyl- bzw. Alkinylreste können entsprechend den Alkylresten substituiert oder durch ein oder mehrere Stickstoffatome unterbrochen sein.

Es ist auch möglich, dass zwei der Reste R' gemeinsam einen Ring bilden, der wiederum mit Alkyl-, Alkenyl- oder Alkinylgruppen substituiert sein kann.

Besonders bevorzugt sind die Reste R' unabhängig voneinander Methyl, Ethyl oder n- oder iso-Propyl.

Unter den aufgeführten Verbindungen sind nicht weiter konjugierte Enamine ausgewählt aus 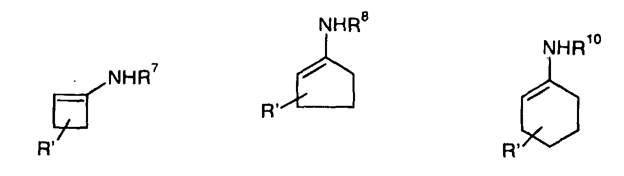 ganz besonders bevorzugt.

Bevorzugt eingesetzte N-heterocyclische Verbindungen in Form von Iminen oder Enaminen sind cyclische Verbindungen mit insgesamt 3 bis 20 -Atomen, bevorzugt 5 bis 12 Atomen, besonders bevorzugt 5 bis 7 Atomen. Dabei kann die N-heterocyclische Verbindung neben dem notwendig vorhandenen Stickstoffatom weitere Heteroatome, bevorzugt Stickstoffatome enthalten. Die Zahl der weiteren Heteroatome ist dabei abhängig von der Ringgröße. Bevorzugt enthalten die N-heterocyclischen Verbindungen ab einer Ringgröße von 5 Atomen kein bis 2 weitere Heteroatome, besonders bevorzugt kein oder 1 weiteres Heteroatom. Die Kohlenstoffatome der N-heterocyclischen Verbindung können weitere Reste tragen. Geeignete Reste sind dabei dieselben, die bereits als Reste R' der Kohlenstoffringe der oben genannten cyclischen Enamine aufgeführt sind. Bei N-heterocyclischen Verbindungen, die neben dem N-Atom weitere Heteroatome, bevorzugt Stickstoffatome, enthalten, können neben den Kohlenstoffatomen auch die Heteroatome, bis auf ein Stickstoffatom, weitere Reste R' tragen. Die N-heterocyclischen Verbindungen können neben der Imin- bzw. Enamin-Doppelbindung weitere Doppelbindungen aufweisen, die zur Imin- bzw. Enamin-Doppelbindung konjugiert oder nicht konjugiert sein können. Bevorzugt werden N-heterocyclische Verbindungen eingesetzt, die keine konjugierten Doppelbindungen aufweisen.

Geeignete N-heterocyclische Verbindungen sind beispielsweise:

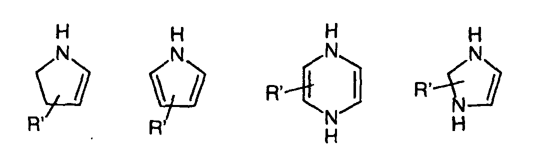

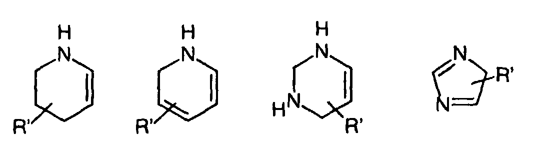

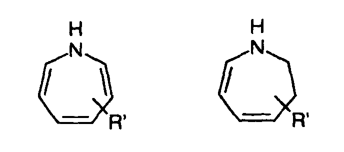

Darin hat der Rest R' dieselbe Bedeutung wie der in Verbindung mit den bevorzugten cyclischen Enaminen genannte Rest R'. Bevorzugt ist R' 0 bis Zahl der Ringatome minus 1, besonders bevorzugt tragen alle Ring-Atome der N-heterocyclischen Verbindungen Wasserstoffatome oder einen Rest R', wobei mindestens ein Stickstoffatom des N-heterocyclischen Rings ein Wasserstoffatom trägt.

Unter den aufgeführten Verbindungen sind N-heterocyclische Verbindungen, die keine konjugierten Doppelbindungen aufweisen, ausgewählt aus 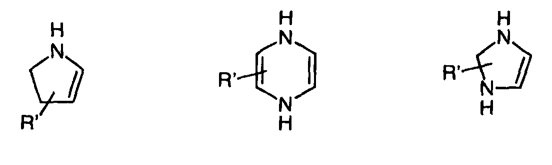 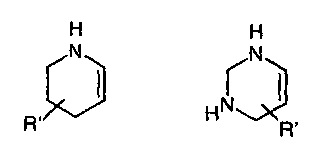 besonders bevorzugt.

Ganz besonders bevorzugt sind Verbindungen der folgenden Formel: 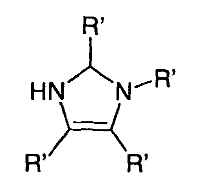 worin R' die obenstehende Bedeutung hat.

Insbesondere bevorzugt ist der Cokatalysator eine Imin- oder tautomere Enaminverbindung, die bei der Dehydrierung der Edukt- und/oder Produktamine gebildet wird oder ein Zerfalls- oder Folgeprodukt der entsprechenden Imin- oder tautomeren Enaminverbindung. Ganz besonders bevorzugt ist der Cokatalysator eine Imin- oder tautomere Enaminverbindung, die bei der Dehydrierung des Eduktamins gebildet wird oder ein Zerfalls- oder Folgeprodukt der Imin- oder tautomeren Enaminverbindung.

Es ist möglich, den gewünschten Cokatalysator herzustellen und separat zu den Ausgangsprodukten zuzugeben. Es ist jedoch auch möglich, den Cokatalysator in-situ vor oder während der Umsetzung (Hydroaminierungsreaktion) herzustellen. Dabei erfolgt die Bildung des Cokatalysators in einer bevorzugten Ausführungsform dadurch, dass man das als Katalysator eingesetzte Hydrid oder Metall vor der Umsetzung mit dem Alken mit dem als Eduktamin gewählten Mono- oder Dialkylamin (oder gegebenenfalls Mono- oder Diarylamin oder Alkylarylamin) umsetzt und den gebildeten Wasserstoff aus dem Reaktionsgemisch entfernt. Bevorzugt erfolgt eine Destillation des Gemisches und anschließende Auftrennung in eine Gasphase, eine das Eduktamin sowie den Cokatalysator enthaltende Leichtsiederfraktion und eine das Hydrid oder das Metall enthaltende Sumpffraktion. Die Leichtsiederfraktion wird anschließend zur Sumpffraktion gegeben, die beschriebene Prozedur optional wiederholt und die Hydroaminierung durch Zugabe von Alken und gegebenenfalls weiterem Eduktamin gestartet.

Es ist auch möglich, den gewünschten Cokatalysator während der Reaktion zu bilden. Die Bildung des Cokatalysators während der Reaktion erfolgt bevorzugt durch Umsetzung der Eduktamine mit Alkenen in Gegenwart eines Metallhydrids oder Metalls als Katalysator, wobei das erhaltene Reaktionsgemisch bevorzugt durch Destillation in eine Gasphase (a) aufgetrennt wird, die Wasserstoff und unumgesetztes Alken enthält, eine Leichtsiederfraktion (b), die nicht umgesetztes Eduktamin und den Cokatalysator enthält, eine Mittelsiederfraktion (c), die Produktamin enthält, und eine Sumpffraktion (d), die den Katalysator enthält. Die Fraktion (b) wird mit frischem Eduktamin und Alken ergänzt und zur Sumpffraktion, die den Katalysator enthält, zurückgefahren. Dabei ist es auch möglich, das nicht umgesetzte Alken aus der Gasphase (a) zu isolieren und gemeinsam mit der Leichtsiederfraktion (b) zur Sumpffraktion zurückzufahren.

In einer bevorzugten Ausführungsform wird das erfindungsgemäße Verfahren so durchgeführt, dass während der Umsetzung entstehender Wasserstoff aus dem Reaktionsgemisch entfernt wird. Dies kann bevorzugt durch Destillation oder Strippung geschehen. Dabei ist zu berücksichtigen, dass die restlichen bei der Destillation abgetrennten Stoffe ganz oder teilweise in das Verfahren zurückgeführt werden.

Ohne an eine Theorie gebunden zu sein, soll im Folgenden ein möglicher Mechanismus, der zur Bildung des Cokatalysators führt, bei der in-situ-Bildung des Cokatalysators vor der Umsetzung mit dem Alken, am Beispiel der Umsetzung von Natriumhydrid und Diethylamin dargestellt werden. Der vorgeschlagene Mechanismus gilt dabei sowohl für den Einsatz von Metallhydriden als auch für den Einsatz von Metallen als Katalysatoren.

Durch die Umsetzung von Natriumhydrid mit Diethylamin werden geringfügige Mengen von NaNEt₂ gebildet. Dieses zerfällt wahrscheinlich nach dem folgenden Schema:

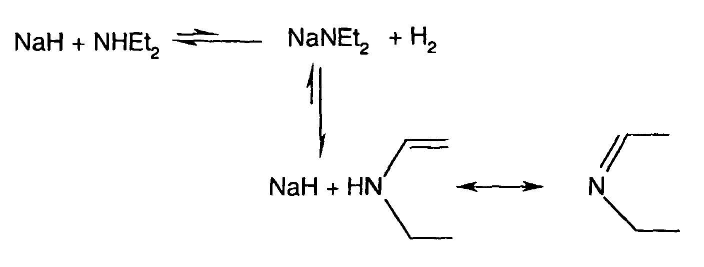

Das dabei entstehende Enamin/Imin besitzt genügend Acidität, die ausreichend ist, um eine Protonierung des Natriumhydrids oder Oxidation des Metalls in hohem Maße zu ermöglichen.

Alternativ oder zusätzlich kann das Imin auch durch Dehydrierung des Eduktamins in Gegenwart eines Hydrier-/Dehydrierkatalysators erfolgen, was im Folgenden Beispiel für Diethylamin als Eduktamin dargestellt ist:

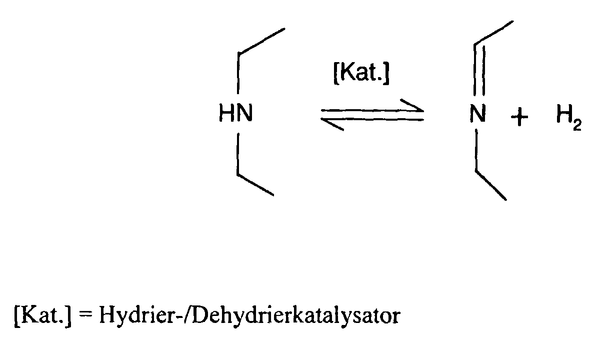

Als Hydrier-/Dehydrierkatalysatoren sind alle üblichen Hydrier-/Dehydrierkatalysatoren geeignet. Im allgemeinen werden Übergangsmetallkatalysatoren in Form von Voll- oder Trägerkatalysatoren eingesetzt. Bevorzugt eingesetzte Übergangsmetalle sind ausgewählt aus den Gruppen VIIIb und Ib des Periodensystems der Elemente. Besonders bevorzugt sind Fe, Ru, Co, Ni, Pd, Pt und Cu oder Legierungen dieser Metalle. Geeignete Trägermaterialien sind z.B. Kohlenstoff, SiO₂, Al₂O₃, ZrO₂ und TiO₂. Des weiteren können die Hydrier-/Dehydrierkatalysatoren Promotoren/Modifier ausgewählt aus Sn, Sb, Alkalimetallen, Erdalkalimetallen, Bi und Pb enthalten. Geeignete Hydrier-/Dehydrierkatalysatoren sind beispielsweise Raney-Ni, Raney-Cu, Raney-Co, Pd/γ-Al₂O₃, Pt/Kohle, Ru/SiO₂, Pd/Sn/Cs/γ-Al₂O₃.

Die genauen Zusammensetzungen geeigneter Hydrier-/Dehydrierkatalysatoren sind dem Fachmann bekannt.

Die Hydrier-/Dehydrierkatalysatoren werden in einer bevorzugten Ausführungsform des erfindungsgemäßen Verfahrens gemeinsam mit dem als Katalysator eingesetzten Metallhydrid oder Metall in einem Reaktor eingesetzt.

In einer bevorzugten Ausführungsform des erfindungsgemäßen Verfahrens wird der Wasserstoff während den vorstehend dargestellten Umsetzungen aus dem System entfernt, um das Gleichgewicht weiter in Richtung der gewünschten Cokatalysatoren zu verschieben.

Ein weiterer Gegenstand der vorliegenden Anmeldung ist somit ein erfindungsgemäßes Verfahren zur Herstellung von Alkylaminen (Produktaminen) durch Umsetzung von Alkenen mit Aminen (Eduktaminen) in Anwesenheit eines Metallhydrids oder Metalls als Katalysator, wobei die Umsetzung in Anwesenheit eines Cokatalysators erfolgt, umfassend die folgenden Schritte:
- Umsetzung des Metallhydrids oder Metalls mit dem Eduktamin gegebenenfalls in Anwesenheit eines Hydrier-/Dehydrierkatalysators,
- Abtrennung einer Gasphase (a) enthaltend Wasserstoff, einer Leichtsiederfraktion (b) enthaltend den Cokatalysator und Eduktamin von einer Sumpffraktion (c) enthaltend das Metallhydrid oder das Metall, bevorzugt durch Destillation,
- Rückführung der Leichtsiederfraktion (b) zur Sumpffraktion (c),
- gegebenenfalls Wiederholung der vorhergegangenen Schritte,
- Umsetzung zu den gewünschten Produktaminen durch Zugabe von Alken und gegebenenfalls weiterem Eduktamin.

In diesem Verfahren erfolgt die Bildung des Cokatalysators in-situ vor der Umsetzung des Eduktamins mit dem Alken (Hydroaminierung). Es ist weiterhin möglich, dass die Bildung des Cokatalysators während der Umsetzung des Eduktamins mit dem Alken erfolgt.

Die Bildung des Cokatalysators in-situ während der Hydroaminierung erfolgt vorzugsweise nach einem Verfahren zur Herstellung von Alkylaminen (Produktaminen) durch Umsetzung von Alkenen mit Aminen (Eduktaminen) in Anwesenheit eines Metallhydrids oder Metalls als Katalysator, wobei die Umsetzung in Anwesenheit eines Cokatalysators erfolgt, umfassend die folgenden Schritte:
- Umsetzung der Alkene mit den Eduktaminen in Anwesenheit des Metallhydrids oder Metalls als Katalysator, gegebenenfalls in Anwesenheit eines Hydrier-/Dehydrierkatalysators
- Auftrennung des Reaktionsgemisches, bevorzugt durch Destillation, in eine Gasphase (a) enthaltend Wasserstoff und nicht umgesetztes Alken, eine Leichtsiederfraktion (b) enthaltend den Cokatalysator und Eduktamin, eine Mittelsiederfraktion (b) enthaltend das gewünschte Alkylamin (Produktamin) und eine das Metallhydrid oder Metall enthaltende Sumpffraktion (c),
- Rückführung der Leichtsiederfraktion (b) zur Sumpffraktion (c),
- Zugabe von frischem Alken und Eduktamin und
- gegebenenfalls Trennung der Gasphase (a) in Wasserstoff und Alken und Rückführung des Alkens gemeinsam mit der Leichtsiederfraktion (b).

Dieses Verfahren kann während des Reaktionsverlaufs mehrmals durchgeführt werden, um frischen Cokatalysator für die Umsetzung bereitzustellen. Des Weiteren ist eine Kombination denkbar, in der zunächst der Cokatalysator in-situ vor der Reaktion gebildet wird, ohne Zugabe des Alkens, und anschließend weiterer Cokatalysator in-situ während der Umsetzung in Anwesenheit des Alkens gebildet wird.

Das erfindungsgemäße Verfahren kann kontinuierlich, diskontinuierlich oder semikontinuierlich (z. B. durch Dosierung von Alken) durchgeführt werden. Die Zugabereihenfolge der Ausgangsprodukte ist dabei im Allgemeinen beliebig und die Zugabe kann entweder gleichzeitig oder nacheinander erfolgen. In einer bevorzugten Ausführungsform wird, der Cokatalysator während der Hydroaminierung (Umsetzung der Alkene mit Aminen (Eduktaminen) in Anwesenheit eines Metallhydrids oder Metalls als Katalysator) gebildet.

Im Allgemeinen erfolgt die Umsetzung bei Temperaturen von 50 bis 200°C, bevorzugt von 70 bis 150°C, besonders bevorzugt von 90 bis 130°C. Der Reaktionsdruck beträgt im Allgemeinen 3 bis 300 bar, bevorzugt 10 bis 100 bar, besonders bevorzugt 20 bis 60 bar. Das bedeutet, zur Durchführung des erfindungsgemäßen Verfahrens sind weder besonders hohe Drucke noch besonders hohe Temperaturen, die besondere Sicherheitsvorkehrungen erfordern, notwendig. Die Reaktionsdauer (Verweilzeit) ist abhängig von den eingesetzten Ausgangssubstanzen und dem gewünschten Umsatzgrad. Sie beträgt im Allgemeinen von 0,2 bis 20 Stunden, bevorzugt von 0,3 bis 10 Stunden, besonders bevorzugt von 0,3 bis 10 Stunden.

Geeignete Vorrichtungen zur Durchführung des erfindungsgemäßen Verfahrens können, je nachdem ob es sich um ein kontinuierliches, diskontinuierliches oder semikontinuierliches Verfahren handelt, verschiedene Reaktortypen wie Blasensäulen (bevorzugt kaskadiert), begaste Rührkessel oder Strahlschlaufenreaktoren oder Kombinationen dieser Reaktoren sein.

Die aus Alkalimetall oder Alkalimetallhydrid und dem Cokatalysator gebildete Alkalimetallverbindung kann homogen gelöst in der Flüssigphase oder bei Überschreiten ihrer Löslichkeit auch neben dem Metall oder Hydrid in Suspension vorliegen.

Bevorzugt werden zur Verhinderung von Anbackungen bestehend aus Alkalimetall, Alkalimetallhydriden und/oder Alkalisalzen Rührkessel bzw. Rührkesselkaskaden mit geeigneten Rührwerken verwendet.

Bei einer diskontinuierlichen Auslegung des Verfahrens werden die gebildeten Additionsprodukte (Hydroaminierungsprodukte) bevorzugt aus dem Reaktor abdestilliert. Der Katalysator, kann - solange er über eine ausreichende Aktivität verfügt - im Reaktor verbleiben und kann so für weitere Umsetzungen genutzt werden.

Bei der kontinuierlichen Auslegung des Verfahrens kann das gebildete Addukt z.B. durch Strippen mit unumgesetzten Alken der Reaktionsmischung entzogen werden. Der Reaktionsaustrag wird jedoch bevorzugt flüssig dem Reaktor entnommen und einer Flashverdampfung oder einer Destillation zugeführt. Alternativ zur thermischen Aufarbeitung des Reaktionsaustrages mit anschließender Rückführung des Katalysators kann zur Katalysatorrückführung bzw. Rückhaltung z. B. eine Filtration eingesetzt werden.

Das molare Verhältnis von Eduktamin zum eingesetzten Alken beträgt im Allgemeinen 20 zu 1 bis 1 zu 10, bevorzugt 10 zu 1 bis 1 zu 5, besonders bevorzugt 2 zu 1 bis 1 zu 2.

Das Metallhydrid oder Metall (Katalysator) wird im Allgemeinen in einer Menge von 0,01 bis 30 Mol.-%, bevorzugt 0,1 bis 10 Mol.-%, besonders bevorzugt 1 bis 3 Mol.-%, bezogen auf das eingesetzte Amin, eingesetzt. Der Cokatalysator liegt im Allgemeinen in einer Menge von 0,002 bis 2 Mol.-% vor , bevorzugt 0,02 bis 1 Mol.-%, besonders bevorzugt 0,05 bis 0,5 Mol.-%, bezogen auf das eingesetzte Amin vor.

Mit dem erfindungsgemäßen Verfahren können sekundäre und tertiäre Amine in Raum-Zeit-Ausbeuten von im Allgemeinen 1 bis 5000 kg/m³h, bevorzugt 10 bis 1000 kg/m³h, besonders bevorzugt 50 bis 200kg/m³h hergestellt werden. Die Ausbeute der gewünschten Amine beträgt - in Abhängigkeit von der Zeit - im Allgemeinen 20 bis 100%, bevorzugt 50 bis 95%, besonders bevorzugt 70 bis 90%, bezogen auf das eingesetzte Amin.

Als Lösungsmittel für die Hydroaminierung eignen sich:
a) Kohlenwasserstoffe, bevorzugt gesättigte Kohlenwasserstoffe mit 2 bis 100 Kohlenstoffatomen. Besonders bevorzugt sind Paraffinöl, n-Butan, i-Butan, n-Pentan, i-Pentan, n-Hexan, i-Hexan und Cyclohexan.
b) tertiäre Amine, bevorzugt Trialkylamine, wobei die Alkylgruppen der Trialkylamine ganz besonders bevorzugt 1 bis 10 Kohlenstoffatome aufweisen. Es sind sowohl lineare und verzweigte Alkylgruppen als auch Cycloalkylgruppen geeignet.

Ganz besonders bevorzugt wird das gewünschte tertiäre Produktamin als Lösungsmittel eingesetzt.

In der diskontinuierlichen Ausführungsform des erfindungsgemäßen Verfahrens werden die getrockneten Eduktamine (z. B. Trocknung über Molekularsieb) zusammen mit einem Metallhydrid, bevorzugt Natriumhydrid oder Kaliumhydrid, oder einem Metall, bevorzugt Natrium oder Kalium, in einen Druckbehälter überführt. Anschließend wird das Reaktionsgemisch auf die gewünschte Temperatur erhitzt und der Gesamtdruck über eine Dosierung des entsprechenden gasförmigen Alkens eingestellt. Nach einer Reaktionszeit von 0,5 bis 20 Stunden, bevorzugt von 1 bis 10 Stunden, besonders bevorzugt von 2 bis 4 Stunden werden die gasförmigen Substanzen (Wasserstoff und nicht umgesetztes Alken) und die Leichtsieder (Cokatalysator und gegebenenfalls nicht umgesetztes Eduktamin) teilweise destillativ abgetrennt. Die Leichtsieder und gegebenenfalls nicht umgesetztes Alken werden in das Reaktionsgemisch zurückgeführt und die Hydroaminierungsreaktion wird weiter fortgesetzt. Nach einer weiteren Reaktionsdauer von 0,1 bis 20 Stunden, bevorzugt von 0,5 bis 10 Stunden, besonders bevorzugt von 1 bis 4 Stunden wird der Destillations-/Rückführungsvorgang gegebenenfalls wiederholt. Im Anschluss daran erfolgt die weitere Hydroaminierungsreaktion, gegebenenfalls unter Zugabe von frischem Eduktamin und frischem Alken. Falls erwünscht, kann der Destillations-/Rückführungsvorgang ein oder mehrere weitere Male wiederholt werden.

Das Produktamin kann bei den jeweiligen Destillationsvorgängen und/oder nach Aufarbeitung des kompletten Reaktorinhalts gewonnen werden.

In der ganz besonders bevorzugten kontinuierlichen Ausführungsform des erfindungsgemäßen Verfahrens wird der Reaktionsaustrag enthaltend 5 bis 60 Gew.-% Eduktamin, 30 bis 95 Gew.-% Produktamin, 0,1 bis 20 Gew.-% Alken und 0,1 bis 10 Gew.-% Metallhydrid oder Metall sowie 0,01 bis 1 Gew.-% Cokatalysator einem Flashbehälter zugeführt. Der Druck im Flashbehälter wird bevorzugt so gewählt, dass sich Temperaturen von 100 bis 150 °C im Flashbehälter ergeben.

Der flüssige Austrag aus dem Flashbehälter enthaltend 60 bis 98,79 Gew.-% Produktamin, 1 bis 30 Gew.-% Eduktamin, 0,2 bis 30 Gew.-% Metallhydrid oder Metall sowie 0,01 bis 0,2 Gew.-% Cokatalysator wird in den Reaktor zurückgeführt. Das in diesem flüssigen Austrag anfallende Produktamin wird kontinuierlich ausgeschleust.

Der überwiegende Teil des Reaktoraustrags wird dem Flashbehälter dampfförmig entnommen und anschließend einer destillativen Aufarbeitung zugeführt.

Die bei der destillativen Aufarbeitung als Kopfprodukte anfallenden Leichtsieder, umfassend das Eduktamin, den Cokatalysator, das Alken sowie Wasserstoff werden - bis auf den Wasserstoff - in den Reaktor zurückgeführt.

Bei dieser Verfahrensweise wird der Cokatalysator überwiegend im Flashbehälter gebildet, da Wasserstoff und Cokatalysator kontinuierlich aus dem Gleichgewicht zwischen Eduktamin und Metallhydrid oder Metall auf der einen und Wasserstoff und Cokatalysator auf der anderen Seite des Gleichgewichts entzogen werden.

Der Flashbehälter stellt somit einen "Cokatalysatorreaktor" dar. Diese Funktion des Flashbehälters kann auch von dem Sumpf einer Kolonne übernommen werden, dem der Reaktionsaustrag direkt zugeführt wird. Der Kolonnendruck wird bevorzugt so eingestellt, dass die Sumpftemperatur 100 bis 150 °C beträgt.

In dem Flashbehälter bzw. in dem Kolonnensumpf kann zusätzlich ein Hydrier/Dehydrierkatalysator enthalten sein.

Ein weiterer Gegenstand der vorliegenden Anmeldung ist die Verwendung eines Cokatalysators bei der Herstellung von Alkylaminen (Produktaminen) durch Umsetzung von Alkenen mit Aminen (Eduktaminen) in Anwesenheit eines Metallhydrids oder Metalls als Katalysator, wobei der Cokatalysator eine ungesättigte Stickstoffverbindung in Form einer cyclischen oder offenkettigen Imin- oder der tautomeren Enaminverbindung ist. Bevorzugte Ausführungsformen des Cokatalysators, des Metallhydrids oder Metalls sowie der Alkene und Eduktamine sind vorstehend angegeben.

Die nachfolgenden Beispiele erläutern die Erfindung zusätzlich.

### Beispiele:

Die Eduktamine wurden über 3Å-Molsieb getrocknet, und zusammen mit handelsüblichem NaH oder KH (Suspension in Paraffinöl, gewaschen mit Diethylamin) in einen 0,1 l Stahlautoklaven überführt. Dann wurde auf 120°C (NaH) bzw. 100°C (KH) erhitzt und über eine Ethylendosierung 50 bar (NaH) bzw. 45 bar (KH) Gesamtdruck eingestellt. Zur Verfolgung der Reaktion wurden aus der Flüssigphase über ein Ventil Proben entnommen, gaschromatographisch analysiert (30m RTX-5 Amine) und über empirisch bestimmte Korrekturfaktoren in Massenprozent umgerechnet.

Die zuerst aufgeführten Vergleichsversuche 1 bis 3 sind Umsetzungen von Diethylamin mit Ethylen in Anwesenheit von Natrium- bzw. Kaliumhydrid als Katalysator in Abwesenheit eines Cokatalysators.

| | | |
|---|---|---|
| *Vergleichsversuch 1: Reaktion von 70 g Diethylamin mit Ethylen an 3 g NaH* | | |

| Versuchszeit [h] | Ausbeute Triethylamin | Ausbeute pro Zeit [1/h] |
|---|---|---|
| 3 | 7% | 2% |
| 4,5 | 12% | 3% |
| 6 | 19% | 5% |
| 7 | 24% | 5% |
| 8 | 31% | 7% |
| 9 | 36% | 5% |
| 10 | 40% | 5% |
| 11 | 43% | 3% |
| 12 | 47% | 4% |
| 13 | 50% | 3% |

| | | |
|---|---|---|
| *Vergleichsversuch 2: Reaktion von 70 g Diethylamin mit Ethylen an 3 g NaH mit Abdestillation der Leichtsieder (Masse des Kondensats: 35g; Zusammensetzung: 0,6% Ethen, 0,15% Imin (N-Ethyliden-Ethylimin), 92,46% Diethylamin, 6,61% Triethylamin) und Ersatz durch Diethylamin nach 1 Stunde Laufzeit und Wiederholung (Masse des Kondensats: 35g; Zusammensetzung: 0,61% Ethen, 0,11% Imin (N-Ethyliden-Ethylimin), 85,65% Diethylamin, 13,29% Triethylamin) nach 3 Stunden Laufzeit.* | | |

| Versuchszeit [h] | Ausbeute Triethylamin | Ausbeute pro Zeit [1/h] |
|---|---|---|
| 1 | 4% | 4% |
| 2 | 8% | 4% |
| 3 | 13% | 5% |
| 4 | 19% | 6% |
| 5,75 | 27% | 5% |
| 6,5 | 31% | 6% |

| | | |
|---|---|---|
| *Vergleichsversuch 3: Reaktion von 70 g Diethylamin mit Ethylen an 3 g KH* | | |

| Versuchszeit [h] | Ausbeute Triethylamin | Ausbeute pro Zeit [1/h] |
|---|---|---|
| 1 | 1% | 1% |
| 2 | 5% | 4% |
| 3 | 12% | 7% |
| 4 | 19% | 8% |
| 5 | 28% | 8% |
| 6 | 38% | 10% |
| 7 | 44% | 6% |
| 8 | 50% | 7% |
| 8,75 | 55% | 6% |

| | | | |
|---|---|---|---|
| *Versuch 1: Reaktion von 70 g Diethylamin mit Ethylen an 3 g NaH mit Abdestillation der Leichtsieder (Masse des Kondensats: 35g; Zusammensetzung: 0,22% Ethen, 0,13% Imin (N-Ethyliden-Ethylimin), 95,59% Diethylamin, 3,92% Triethylamin) und Rückführung nach 1 Stunde Laufzeit und Wiederholung (Masse des Kondensats: 35g; Zusammensetzung: 0,2% Ethen, 0,17% lmin (N-Ethyliden-Ethylimin), 70,41% Diethylamin, 29,01% Triethylamin) nach 3 Stunden Laufzeit*. | | | |

| Versuchszeit [h] | Ausbeute Triethylamin | Ausbeute pro Zeit [1/h] | Kommentar |
|---|---|---|---|
| 1 | 3% | 3% | |
| 2 | 16% | 14% | hohe Aktivität durch Rückführung |
| 3 | 25% | 9% | |
| 4,2 | 42% | 15% | hohe Aktivität durch Rückführung |
| 5 | 50% | 10% | |
| 6 | 57% | 7% | |

| | | | |
|---|---|---|---|
| *Versuch 2: Reaktion von 70 g Diethylamin mit Ethylen an 3 g KH mit Abdestillation der Leichtsieder (Masse des Kondensats: 35g; Zusammensetzung: 0,45% Ethen, 0,06% Imin (N-Ethyliden-Ethylimin), 77,88% Diethylamin, 21,41% Triethylamin) und Rückführung zu Versuchsbeginn.* | | | |

| Versuchszeit [h] | Ausbeute Triethylamin | Ausbeute pro Zeit [1/h] | Kommentar |
|---|---|---|---|
| 1 | 7% | 7% | hohe Aktivität durch Rückführung |
| 2,17 | 17% | 9% | |
| 3 | 24% | 8% | |

| | | | |
|---|---|---|---|
| *Versuch 3: Reaktion von 68,5 g Diethylamin und 1,5 g N-Ethylidenethylimin mit Ethylen an 3 g NaH mit Abdestillation der Leichtsieder (Masse des Kondensats: 35g; Zusammensetzung: 0,2% Ethen, 0,02% Imin (N-Ethyliden-Ethylimin), 80,15% Diethylamin, 19,41 % Triethylamin) und Rückführung nach 1 Stunde Laufzeit.* | | | |

| Versuchszeit [h] | Ausbeute Triethylamin | Ausbeute pro Zeit [1/h] | Kommentar |
|---|---|---|---|
| 1 | 13% | 13% | hohe Aktivität durch Imin |
| 2 | 29% | 16% | hohe Aktivität durch Rückführung |

Man erkennt deutlich, dass die Aktivität des Alkalimetallhydrids steigt, wenn zuvor abdestilliert und das Destillat zurückgefahren wird. Die Reaktion verlangsamt sich schließlich wieder durch Desaktivierungsvorgänge am Katalysator und kann durch erneutes Abdestillieren und Zurückfahren wieder beschleunigt werden. Andererseits erhält man die hohe Aktivität des Alkalihydrids schon zu Beginn des Versuches, wenn man einige Gewichtsprozent N-Ethylidenethylimin dem Diethylamin als Cokatalysator zufügt.

Dies lässt den Schluss zu, dass - ohne an eine Theorie gebunden zu sein - durch das Abdestillieren und Rückführen letztendlich Diethylamin in N-Ethylidenethylimin und H₂ zerfällt und das gebildete H₂ aus dem System entfernt wird. Diese Hypothese wird durch den folgenden Versuch untermauert:

| | | |
|---|---|---|
| *Versuch 4: Vorbehandlung von 70 g Diethylamin und 3 g NaH mit 50 bar H*_{*2*} *bei 120°C, dann abkühlen, entspannen, aufpressen von 50 bar Ethylen und Reaktion mit Ethylen bei 120°C*. | | |

| Versuchszeit [h] | Ausbeute Triethylamin | Ausbeute pro Zeit [1/h] |
|---|---|---|
| 3 | 0,3% | 0,1% |
| 6 | 0,7% | 0,1% |
| 10 | 1,1% | 0,1% |
| 13 | 1,4% | 0,1% |

## Patentansprüche

1. Verfahren zur Herstellung von Alkylaminen (Produktaminen) durch Umsetzung von Alkenen mit Aminen (Eduktaminen) in Anwesenheit eines Metallhydrids oder Metalls als Katalysator, **dadurch gekennzeichnet, dass** die Umsetzung in Anwesenheit eines Cokatalysators erfolgt, der eine ungesättigte Stickstoffverbindung in Form einer cyclischen oder offenkettigen Imin- oder der tautomeren Enamingverbindung ist.

2. Verfahren nach Anspruch 1, **dadurch gekennzeichnet, dass** die Alkene nicht aktivierte Alkene sind, deren olefinische Doppelbindung nicht in Konjugation mit anderen olefinischen oder aromatischen Doppelbindungen bzw. Heteroatomen steht.

3. Verfahren nach Anspruch 1 oder 2, **dadurch gekennzeichnet, dass** die eingesetzten Amine (Eduktamine) primäre oder sekundäre Alkylamine sind.

4. Verfahren nach einem der Ansprüche 1 bis 3, **dadurch gekennzeichnet, dass** die hergestellten Amine (Produktamine) sekundäre oder tertiäre Alkylamine sind.

5. Verfahren nach einem der Ansprüche 1 bis 4, **dadurch gekennzeichnet, dass** der Cokatalysator eine Imin- oder tautomere Enaminverbindung ist, die bei der Dehydrierung des eingesetzten Amins (Eduktamin) gebildet wird oder ein Zerfalls- oder Folgeprodukt der Imin- oder tautomeren Enaminverbindung ist.

6. Verfahren nach Anspruch 5, **dadurch gekennzeichnet, dass** der Cokatalysator in-situ vor oder während der Umsetzung des Eduktamins mit dem Alken gebildet wird.

7. Verfahren nach einem der Anspruch 6, **dadurch gekennzeichnet, dass** die Umsetzung bei einer Reaktionstemperatur von 50 bis 200°C durchgeführt wird.

8. Verfahren nach einem der Ansprüche 1 bis 7, **dadurch gekennzeichnet, dass** der Reaktionsdruck bei der Umsetzung 2 bis 300 bar beträgt.

9. Verwendung eines Cokatalysators, der eine ungesättigte Stickstoffverbindung in Form einer cyclischen oder. offenkettigen Imin- oder der tautomeren Enaminverbindung ist, bei der Herstellung von-Alkylaminen (Prokuktaminen) durch Umsetzung von Alkenen mit Aminen (Eduktaminen) in Anwesenheit eines Metallhydrids oder Metalls als Katalysator.

## Claims

1. A process for preparing alkylamines (product amines) by reacting alkenes with amines (starting amines) in the presence of a metal hydride or metal as catalyst, wherein the reaction is carried out in the presence of a cocatalyst which is an unsaturated nitrogen compound in the form of a cyclic or acyclic imine or of the tautomeric enamine compound.

2. The process according to claim 1, wherein the alkenes are nonactivated alkenes whose olefinic double bond is not conjugated with other olefinic double bonds or heteroatoms.

3. The process according to claim 1 or 2, wherein the amine used (starting amines) are primary or secondary alkylamines.

4. The process according to any of claims 1 to 3, wherein the amines prepared (product amines) are secondary or tertiary alkylamines.

5. The process according to any of claims 1 to 4, wherein the cocatalyst is an imine or tautomeric enamine compound which is formed in the dehydrogenation of the amine used (starting amine) or is a decomposition product or reaction product of the imine or tautomeric enamine compound.

6. The process according to claim 5, wherein the cocatalyst is formed in situ before or during the reaction of the starting amine with the alkene.

7. The process according to claim 6, wherein the reaction is carried out at from 50 to 200°C.

8. The process according to any of claims 1 to 7, wherein the pressure during the reaction is from 2 to 300 bar.

9. The use of a cocatalyst which is an unsaturated nitrogen compound in the form of a cyclic or acyclic imine or of the tautomeric enamine compound in the preparation of alkylamines (product amines) by reacting alkenes with amines (starting amines) in the presence of a metal hydride or metal as catalyst.

## Revendications

1. Procédé de préparation d'alkylamines (amines produit) par réaction d'alcènes avec des amines (amines de départ) en présence d'un hydrure métallique ou d'un métal comme catalyseur, **caractérisé en ce que** la réaction a lieu en présence d'un cocatalyseur qui est un composé azoté insaturé sous la forme d'un composé d'imine cyclique ou à chaîne ouverte ou du composé d'énamine tautomère.

2. Procédé suivant la revendication 1, **caractérisé en ce que** les alcènes sont des alcènes non activés dont la double liaison oléfinique n'est pas en conjugaison avec d'autres doubles liaisons oléfiniques ou aromatiques ou respectivement des hétéroatomes.

3. Procédé suivant la revendication 1 ou 2, **caractérisé en ce que** les amines mises en oeuvre (amines de départ) sont des alkylamines primaires ou secondaires.

4. Procédé suivant l'une des revendications 1 à 3, **caractérisé en ce que** les amines préparées (amines produit) sont des alkylamines secondaires ou tertiaires.

5. Procédé suivant l'une des revendications 1 à 4, **caractérisé en ce que** le cocatalyseur est un composé d'imine ou d'énamine tautomère qui est formé lors de la déshydrogénation de l'amine mise en oeuvre (amine de départ) ou est un produit de décomposition ou un dérivé du composé d'imine ou d'énamine tautomère.

6. Procédé suivant la revendication 5, **caractérisé en ce que** le cocatalyseur est formé in situ avant ou pendant la réaction de l'amine de départ avec l'alcène.

7. Procédé suivant la revendication 6, **caractérisé en ce que** la réaction est effectuée à une température réactionnelle de 50 à 200°C.

8. Procédé suivant l'une des revendications 1 à 7, **caractérisé en ce que** la pression réactionnelle au cours de la réaction est de 2 à 300 bars.

9. Utilisation d'un cocatalyseur, qui est un composé azoté insaturé sous la forme d'un composé d'imine cyclique ou à chaîne ouverte ou du composé d'énamine tautomère, au cours de la préparation d'alkylamines (amines produit) par réaction d'alcènes avec des amines (amines de départ) en présence d'un hydrure métallique ou d'un métal comme catalyseur.
